(19) Europäisches Patentamt
European Patent Office
Office européen des brevets

(11) Veröffentlichungsnummer: **0 391 188 A1**

(12) # EUROPÄISCHE PATENTANMELDUNG

(21) Anmeldenummer: **90105656.4**

(51) Int. Cl.[5]: **C07D 277/68, A01N 43/78**

(22) Anmeldetag: **24.03.90**

(30) Priorität: **07.04.89 DE 3911226**

(43) Veröffentlichungstag der Anmeldung: **10.10.90 Patentblatt 90/41**

(84) Benannte Vertragsstaaten: **BE CH DE FR GB IT LI NL**

(71) Anmelder: **BAYER AG**
**D-5090 Leverkusen 1 Bayerwerk(DE)**

(72) Erfinder: **Wagner, Klaus, Dr.**
**Jakob-Böhme-Strasse 2**
**D-5000 Köln 80(DE)**
Erfinder: **Hänssler, Gerd, Dr.**
**Am Arenzberg 58a**
**D-5090 Leverkusen 3(DE)**
Erfinder: **Brandes, Wilhelm, Dr.**
**Eichendorffstrasse 3**
**D-5653 Leichlingen 1(DE)**

(54) **Fungizide Mittel auf Basis von teilweise bekannten nitrosubstituierten Benzthiazolonen, neue nitrosubstituierte Benzthiazolone und Verfahren zu ihrer Herstellung.**

(57) Fungizide Verwendung von teilweise bekannten nitrosubstituierten Benzthiazolonen der allgemeinen Formel (I)

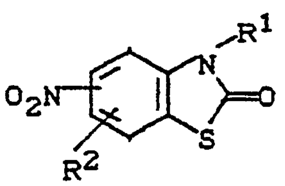

(I)

in welcher
$R^1$ und $R^2$ die in der Beschreibung gegebenen Bedeutungen haben.

Die neuen und bekannten Verbindungen der Formel (I), können nach Analogieverfahren hergestellt werden, z. B. indem man geeignete Benzthiazolone mit geeigneten Halogenverbindungen umsetzt oder geeignete Benzthiazol-3-oxide mit Phosphoroxychlorid umsetzt. Auch einige Zwischenprodukte sind neu und können ebenfalls nach Analogieverfahren hergestellt werden.

EP 0 391 188 A1

## Fungizide Mittel auf Basis von teilweise bekannten nitrosubstituierten Benzthiazolonen, neue nitrosubstituierte Benzthiazolone und Verfahren zu ihrer Herstellung

Die vorliegende Erfindung betrifft die Verwendung von teilweise bekannten nitrosubstituierten Benzthiazolonen als Fungizide, neue nitrosubstituierte Benzthiazolone und Verfahren zu ihrer Herstellung.

Es ist bereits bekannt, daß man bestimmte Pflanzenkrankheiten durch cyclische Schwefelverbindungen bekämpfen kann. So kann z.B. 6-Methyl-2,3-chinoxalindithiol-cyclocarbonat (Chinomethionat/Morestan) gegen Mehltau im Obstbau verwendet werden (vgl. DE-AS 1 100 372). Die Wirkung dieser bekannten Verbindung ist jedoch, insbesondere bei niedrigen Wirkstoffkonzentrationen, nicht immer zufriedenstellend.

Weiter sind durch Nitro- und/oder Trifluormethyl-substituierte Benzthiazolone bekannt geworden (vgl. Chem. Ber. 107 (1974), 305-315; DE-OS 2 101 150), die ebenfalls eine biologische Wirkung haben.

Es wurde nun gefunden, daß die teilweise bekannten nitrosubstituierten Benzthiazolone der allgemeinen Formel (I)

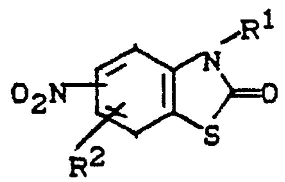

(I)

in welcher

$R^1$ für Wasserstoff, für Alkyl, Alkenyl oder Alkinyl, für Cycloalkyl-alkyl, für Furylalkyl, für Alkylcarbonyl, welches gegebenenfalls durch Halogen oder Alkoxy substituiert ist, für Benzoyl, welches gegebenenfalls durch Halogen, Nitro, Alkyl, Halogenalkyl und/oder Alkoxy substituiert ist, für Alkoxycarbonyl, für Phenoxycarbonyl, für Alkylaminocarbonyl, welches gegebenenfalls durch Halogen oder Alkoxy substituiert ist, für Dialkylaminocarbonyl, für Benzyloxycarbonyl, für Benzylaminocarbonyl, welches gegebenenfalls durch Halogen, Alkyl, Halogenalkyl und/oder Alkoxy substituiert ist, oder für Phenethylaminocarbonyl, welches gegebenenfalls durch Halogen, Alkyl, Halogenalkyl und/oder Alkoxy substituiert ist, steht und

$R^2$ für Wasserstoff, Halogen Alkyl oder Halogenalkyl steht,

starke fungizide Eigenschaften aufweisen.

Überraschenderweise zeigen die nitrosubstituierten Benzthiazolone der allgemeinen Formel (I) erheblich stärkere fungizide Wirkung als strukturell ähnliche Fungizide mit vergleichbarem Wirkprofil, wie z.B. Morestan.

Aliphatische Reste wie Alkyl, allein oder in Zusammensetzungen, sind geradkettig oder verzweigt, auch wenn es nicht ausdrücklich erwähnt ist.

Halogen, allein oder in Zusammensetzungen wie Halogenalkyl, bedeutet Fluor, Chlor, Brom, Iod, vorzugsweise Fluor, Chlor oder Brom.

Die Erfindung betrifft vorzugsweise die Verwendung fungizider Mittel auf Basis von Verbindungen der allgemeinen Formel (I), in welcher

$R^1$ für Wasserstoff, für $C_1$-$C_{10}$-Alkyl, $C_2$-$C_{10}$-Alkenyl, $C_2$-$C_{10}$-Alkinyl, für Cyclohexylmethyl, für Furylmethyl, für $C_1$-$C_6$-Alkyl-carbonyl, welches gegebenenfalls ein- bis fünffach, gleich oder verschieden durch Fluor, Chlor oder $C_1$-$C_4$-Alkoxy substituiert ist, für Benzoyl, welches gegebenenfalls durch 1 bis 3,gleiche oder verschiedene Reste aus der Reihe Fluor, Chlor, Brom, Nitro, Methyl, Ethyl, Trifluormethyl, Methoxy und Ethoxy substituiert ist, für $C_1$-$C_6$-Alkoxy-carbonyl, für Phenoxycarbonyl, für $C_1$-$C_6$-Alkylamino-carbonyl, welches gegebenenfalls ein-bis dreifach, gleich oder verschieden durch Fluor, Chlor oder $C_1$-$C_4$-Alkoxy substituiert ist, für Di-($C_1$-$C_4$-alkyl)-amino-carbonyl, für Benzyloxy-carbonyl, für Benzylaminocarbonyl, welches gegebenenfalls ein- bis dreifach, gleich oder verschieden durch Fluor, Chlor, Brom, Methyl, Ethyl, Trifluormethyl, Methoxy und Ethoxy substituiert ist, oder für Phenethylaminocarbonyl, welches gegebenenfalls ein- bis dreifach, gleich oder verschieden durch Fluor, Chlor, Brom, Methyl, Ethyl, Trifluormethyl, Methoxy und Ethoxy substituiert ist, steht und

$R^2$ für Wasserstoff, Halogen, $C_1$-$C_4$-Alkyl oder $C_1$-$C_4$-Halogenalkyl mit 1 bis 9 gleichen oder verschiedenen Halogenatomen steht.

Besonders bevorzugt werden erfindungsgemäß die Verbindungen der Formel (I) verwendet, in welcher

$R^1$ für Wasserstoff, für Methyl, Ethyl, Propyl, Isopropyl, Butyl, Isobutyl, Pentyl, Isopentyl, Hexyl oder Isohexyl, für Allyl oder Propargyl, für Acetyl, Propionyl, n- und iso-Butyroyl, n-, iso-, sec.- und tert.-Butyl-carbonyl, n-, iso-, sec.- und tert.-Pentyl-carbonyl, n-, iso-, sec.- und tert.-Hexyl-carbonyl, Trifluoracetyl, Chloracetyl, Dichloracetyl, Trichloracetyl, Bromacetyl, α- und β-Chlorpropionyl, Methoxyacetyl, Ethoxyacetyl, für Benzoyl, welches gegebenenfalls durch 1 oder 2 gleiche oder verschiedene Reste aus der Reihe Fluor, Chlor, Brom, Nitro und Methyl substituiert ist, für Methoxycarbonyl, Ethoxycarbonyl, n- oder iso-Propoxycarbonyl, n-, iso- oder sec.-Butoxycarbonyl, für Methylaminocarbonyl, Ethylaminocarbonyl, n- oder iso-Propylamino-carbonyl, n-, iso-, sec.- oder tert.-Butylamino-carbonyl, Pentylaminocarbonyl, für Dimethylaminocarbonyl oder Diethylaminocarbonyl, für Benzyloxycarbonyl, für Benzylaminocarbonyl, welches gegebenenfalls durch 1 oder 2 gleiche oder verschiedene Reste aus der Reihe Fluor, Chlor, Methyl, Trifluormethyl und Methoxy substituiert ist, oder für Phenethylaminocarbonyl, welches gegebenenfalls durch 1 oder 2 gleiche oder verschiedene Reste aus der Reihe Fluor, Chlor Methyl, Trifluormethyl und Methoxy im Phenyl- und/oder Alkylrest substituiert ist, steht und

$R^2$ für Chlor, Methyl oder Trifluormethyl steht.

Beispiele für die erfindungsgemäß zu verwendenden Verbindungen der Formel (I) sind in der nachstehenden Tabelle 1 aufgeführt.

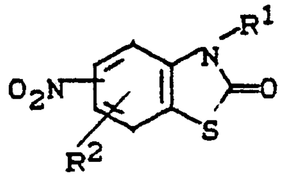 . (I)

Tabelle 1

Beispiele für die Verbindungen der Formel (I)

| Beispiel Nr. | Position von $NO_2$ | $R^1$ | (Position-) $R^2$ | Schmelzpunkt (°C) |
|---|---|---|---|---|
| 1 | 7 | $-CO-CH_3$ | $(5-)CH_3$ | 142 |
| 2 | 7 | $-COOC_2H_5$ | $(5-)CH_3$ | 140 |
| 3 | 5 | H | $(7-)CH_3$ | 250 |
| 4 | 7 | $-CO-NH(CH_2)_2-$ 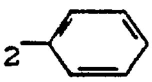 | $(5-)CF_3$ | 143 |
| 5 | 5 | $-CO-CH_3$ | $(7-)CH_3$ | 127 |
| 6 | 7 | $-CO-CH_3$ | $(5-)CF_3$ | 157 |
| 7 | 5 | $-CO-NH(CH_2)_2-$ 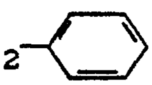 | (7-)Cl | 230 |
| 8 | 5 | $-CO-NHCH_2-$ 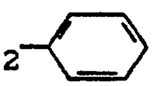 | $(7-)CH_3$ | 156 |
| 9 | 5 | $-CO-NH(CH_2)_2-$ 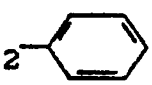 | $(7-)CH_3$ | 151 |

Tabelle 1 (Fortsetzung)

| Beispiel Nr. | Position von $NO_2$ | $R^1$ | (Position-) $R^2$ | Schmelzpunkt (° C) |
|---|---|---|---|---|
| 10 | 5 | $-CO-NHCH_2CH$ 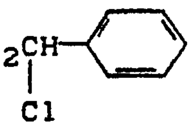 | $(7-)CH_3$ | 155 |
| 11 | 7 | H | $(5-)CH_3$ | |
| 12 | 5 | $-CO-C(CH_3)_3$ | (7-)Cl | |
| 13 | 7 | $-CO-CH_3$ | (5-)Cl | 147 |
| 14 | 7 | $-CO-C_2H_5$ | (5-)Cl | 133 |
| 15 | 5 | $-CO-C_2H_5$ | $(7-)CH_3$ | 156 |
| 16 | 7 | $-CO-C_2H_5$ | $(5-)CH_3$ | 147 |
| 17 | 7 | $-COOCH_2CH(CH_3)_2$ | $(5-)CH_3$ | 87 |
| 18 | 7 | $-CO-C(CH_3)_3$ | $(5-)CH_3$ | 77 |
| 19 | 5 | $CH_3$ | $(7-)CH_3$ | 191 |
| 20 | 7 | $CH_3$ | $(5-)CH_3$ | 216 |
| 21 | 5 | $-CO-NH(CH_2)_2CH(CH_3)_2$ | (7-)Cl | 97 |
| 22 | 7 | $-CO-NH(CH_2)_2CH(CH_3)_2$ | (5-)Cl | 101 |
| 23 | 7 | $-CO-NH(CH_2)_2$ 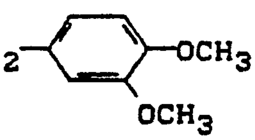 | (5-)Cl | 125 |
| 24 | 7 | $-CO-NHCH_2$ 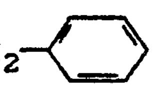 | (5-)Cl | 110 |
| 25 | 7 | $CH_3$ | (5-)Cl | 172 |
| 26 | 5 | $-CO-NHCH(CH_3)_2$ | (7-)Cl | 110 |

Tabelle 1 (Fortsetzung)

| Beispiel Nr. | Position von $NO_2$ | $R^1$ | (Position-) $R^2$ | Schmelzpunkt (°C) |
|---|---|---|---|---|
| 27 | 5 | $-CO-NHCH_2-$ 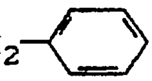 | (7-)Cl | 113 |
| 28 | 5 | $-CO-NH(CH_2)_2-$ 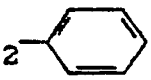 | (7-)$CF_3$ | 106 |
| 29 | 7 | $-CO-NHCH_2-$ 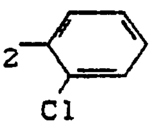 | (5-)$CF_3$ | 131 |
| 30 | 7 | $-CO-NH(CH_2)_2CH(CH_3)_2$ | (5-)$CF_3$ | 74 |
| 31 | 7 | $-CO-NHCH_2-$ 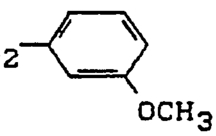 | (5-)$CF_3$ | 122 |
| 32 | 7 | $-CO-NHCH_2-$ 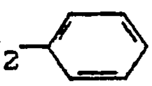 | (5-)$CF_3$ | 115 |
| 33 | 5 | $-CO-CH(CH_3)_2$ | (7-)$CH_3$ | 143 |
| 34 | 5 | $CH_3$ | (7-)$CF_3$ | 126 |
| 35 | 5 | $-CO-C_2H_5$ | (7-)$CF_3$ | 106 |
| 36 | 5 | $-CO-CH_2CH(CH_3)_2$ | (7-)$CF_3$ | 105 |
| 37 | 5 | $CH_3$ | (7-)Cl | 176 |
| 38 | 7 | $-CO-C_2H_5$ | (5-)$CF_3$ | 100 |
| 39 | 7 | $-CO-(CH_2)_5-CH_3$ | (5-)$CF_3$ | 72 |
| 40 | 7 | $-CO-(CH_2)_5-CH_3$ | (5-)Cl | 80 |

<u>Tabelle 1</u> (Fortsetzung)

| Beispiel Nr. | Position von $NO_2$ | $R^1$ | (Position-) $R^2$ | Schmelzpunkt (°C) |
|---|---|---|---|---|
| 41 | 5 | $-CO-(CH_2)_5-CH_3$ | $(7-)CF_3$ | 93 |
| 42 | 5 | $-CO(CH_2)_5-CH_3$ | $(7-)CH_3$ | 74 |
| 43 | 7 | $-CO-NH(CH_2)_2-$ 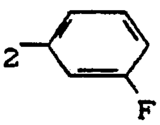 | $(5-)CF_3$ | 128 |
| 44 | 7 | $-CO-NH(CH_2)_2-$ 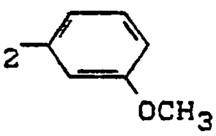 | $(5-)CF_3$ | 120 |
| 45 | 5 | $-CO-NHCH_2-$ 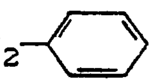 | $(7-)CF_3$ | 116 |
| 46 | 7 | $CH_3$ | $(5-)CF_3$ | |
| 47 | 5 | $-CO-NHCH_2-$ 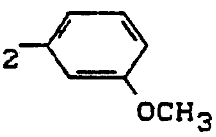 | $(7-)CF_3$ | 110 |
| 48 | 5 | $-CO-CH_3$ | $(7-)Cl$ | 126 |
| 49 | 5 | $-CO-(CH_2)_5-CH_3$ | $(7-)Cl$ | 78 |
| 50 | 7 | $-CO-NHCH_2-$ 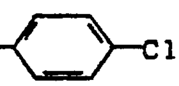 | $(5-)CF_3$ | 130 |
| 51 | 7 | $-CO-NHCH_2-$ 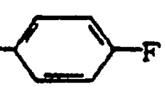 | $(5-)CF_3$ | 142 |
| 52 | 5 | $-CO-NH(CH_2)_2-$ 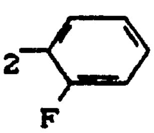 | $(7-)CF_3$ | 121 |

Tabelle 1 (Fortsetzung)

| Bei-spiel Nr. | Posi-tion von $NO_2$ | $R^1$ | (Position-) $R^2$ | Schmelz-punkt (°C) |
|---|---|---|---|---|
| 53 | 7 | $-CO-NH(CH_2)_2-$ 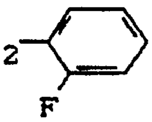 | (5-)$CF_3$ | 134 |
| 54 | 5 | $-CO-NH(CH_2)_2-$ 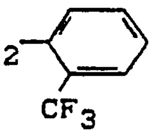 | (7-)$CF_3$ | 106 |
| 55 | 7 | $-CO-NH(CH_2)_2-$ 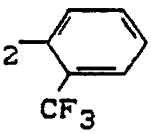 | (5-)$CF_3$ | 129 |
| 56 | 7 | $-CO-(CH_2)_5-CH_3$ | (5-)$CH_3$ | 97 |
| 57 | 5 | $-CO-C_2H_5$ | (7-)Cl | 137 |
| 58 | 6 | $-CO-C_2H_5$ | (7-)Cl | 85 |
| 59 | 7 | $-CO-CH(CH_3)_2$ | (5-)$CH_3$ | 97 |
| 60 | 6 | $-CO-CH_3$ | (7-)Cl | 129 |
| 61 | 6 | $-CO-(CH_2)_5-CH_3$ | (7-)Cl | 30 |
| 62 | 7 | $-CO-NHCH_2-$ 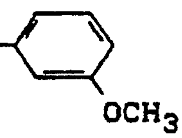 | (5-)$CH_3$ | 138 |
| 63 | 7 | $-CO-NHCH_2-$ 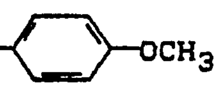 | (5-)$CH_3$ | 160 |
| 64 | 7 | $-CO-NH(CH_2)_2-$ 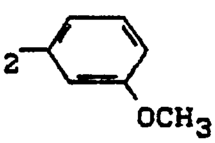 | (5-)$CH_3$ | 135 |

Tabelle 1 (Fortsetzung)

| Beispiel Nr. | Position von $NO_2$ | $R^1$ | (Position-) $R^2$ | Schmelzpunkt (° C) |
|---|---|---|---|---|
| 65 | 7 | $-CO-NHCH_2-$ 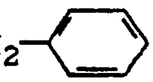 | (5-)$CH_3$ | 138 |
| 66 | 7 | $-CO-NH(CH_2)_2-$ 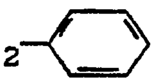 | (5-)$CH_3$ | 153 |
| 67 | 6 | $-CO-NHCH_2-$ 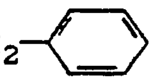 | (7-)Cl | 124 |
| 68 | 7 | $-CO-NHCH_2-$ 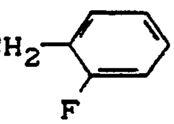 | (5-)$CF_3$ | 106 |
| 69 | 7 | $-CO-NHCH_2-$ 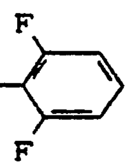 | (5-)$CF_3$ | 128 |
| 70 | 7 | $-CO-NH(CH_2)_2-$ 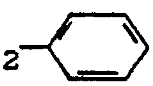 | (5-)Cl | 131 |
| 71 | 7 | $-CO-NHCH_2-$ 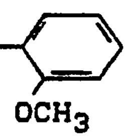 | (5-)Cl | 157 |
| 72 | 7 | $-CO-NHCH_2-$ 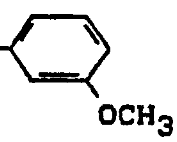 | (5-)Cl | 144 |
| 73 | 7 | $-CO-NHCH_2-$ 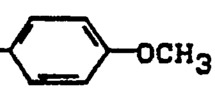 | (5-)Cl | 145 |

<u>Tabelle 1</u> (Fortsetzung)

| Beispiel Nr. | Position von $NO_2$ | $R^1$ | (Position-) $R^2$ | Schmelzpunkt (°C) |
|---|---|---|---|---|
| 74 | 7 | $-CO-NH(CH_2)_2-$ 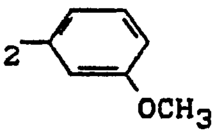 | (5-)Cl | 125 |
| 75 | 7 | $-CO-NHCH_2-$ 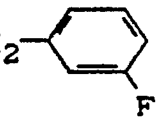 | (5-)$CF_3$ | 100 |
| 76 | 5 | $C_2H_5$ | (7-)$CF_3$ | 112 |
| 77 | 5 | $-CH_2-CH=CH_2$ | (7-)$CF_3$ | 104 |
| 78 | 7 | -COO- 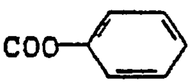 | (5-)$CH_3$ | 152 |
| 79 | 6 | CO- 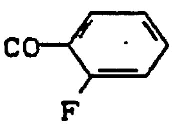 | - | 174 |
| 80 | 6 | $-COCH_2CH(CH_3)_2$ | (7-)Cl | 68 |
| 81 | 6 | $-CO-CH(CH_3)_2$ | (7-)Cl | 106 |
| 82 | 6 | $-CO-(CH_2)_5-CH_3$ | - | 80 |
| 83 | 5 | H | (7-)Cl | 248 |
| 84 | 7 | -CO- 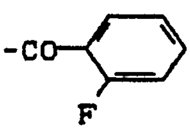 | (5-)$CH_3$ | 180 |
| 85 | 7 | -CO- 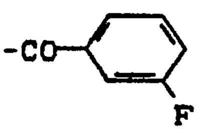 | (5-)$CH_3$ | 156 |

<u>Tabelle 1</u> (Fortsetzung)

| Beispiel Nr. | Position von $NO_2$ | $R^1$ | (Position-) $R^2$ | Schmelzpunkt (°C) |
|---|---|---|---|---|
| 86 | 7 | -CO 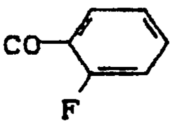 | (5-)Cl | 192 |
| 87 | 7 | -CO 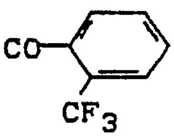 | (5-)$CH_3$ | 149 |
| 88 | 7 | -CO 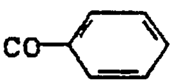 | (5-)Cl | 156 |
| 89 | 7 | -CO 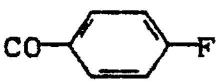 | (5-)Cl | 150 |
| 90 | 7 | -CO 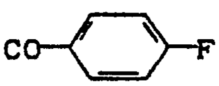 | (5-)$CH_3$ | 188 |
| 91 | 6 | -CO 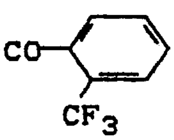 | (7-)Cl | 163 |
| 92 | 5 | $-CO-NH-CH_2$ 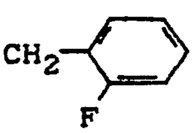 | (7-)$CF_3$ | 130 |
| 93 | 5 | $-CO-NH-CH_2$ 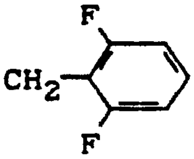 | (7-)$CF_3$ | 150 |

Tabelle 1 (Fortsetzung)

| Beispiel Nr. | Position von $NO_2$ | $R^1$ | (Position-) $R^2$ | Schmelzpunkt (°C) |
|---|---|---|---|---|
| 94 | 5 | 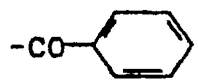 | (7-)$CH_3$ | 201 |
| 95 | 5 | 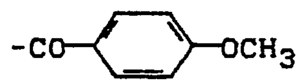 | (7-)$CH_3$ | 210 |
| 96 | 5 | 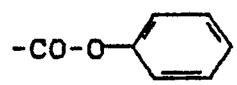 | (7-)$CH_3$ | 159 |
| 97 | 5 | 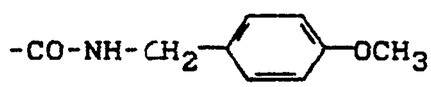 | (7-)Cl | 178 |
| 98 | 6 | 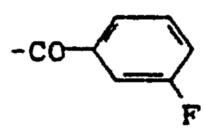 | (7-)Cl | (amorph) |
| 99 | 7 | 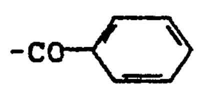 | (5-)$CH_3$ | 179 |
| 100 | 5 | 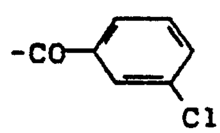 | (7-)$CH_3$ | 210 |
| 101 | 5 | 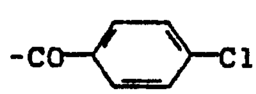 | (7-)$CH_3$ | 195 |

Tabelle 1 (Fortsetzung)

| Beispiel Nr. | Position von $NO_2$ | $R^1$ | (Position-) $R^2$ | Schmelzpunkt (°C) |
|---|---|---|---|---|
| 102 | 5 | 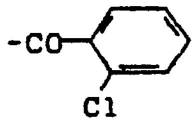 | (7-)$CH_3$ | 210 |
| 103 | 5 | 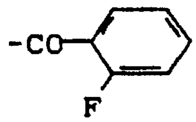 | (7-)$CH_3$ | 179 |
| 104 | 5 | 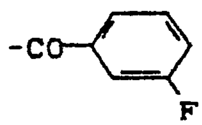 | (7-)$CH_3$ | 181 |
| 105 | 5 | 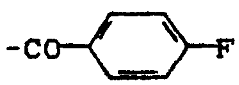 | (7-)$CH_3$ | 199 |
| 106 | 5 | 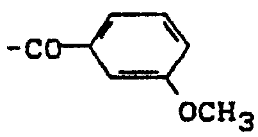 | (7-)$CH_3$ | 167 |
| 107 | 5 | 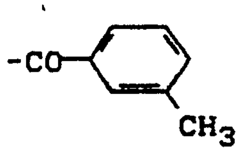 | (7-)$CH_3$ | 181 |
| 108 | 5 | 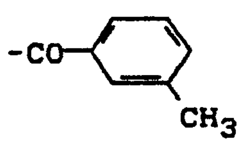 | (7-)$CF_3$ | 155 |

Die Verbindungen der Formel (I) sind zum Teil bekannt oder können nach an sich bekannten Verfahren hergestellt werden (vgl. Chem. Ber. 107 (1974), 305-316; Egypt. J. Chem. 16 (1973), 355-359; JP-A 60-105 671; DE-OS 2 101 150; EP-A 218 972).

Gegenstand der vorliegenden Erfindung sind weiterhin neue nitrosubstituierte Benzthiazolone der allgemeinen Formel (Ia)

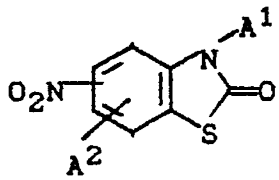

(Ia)

in welcher

$A^1$ für Alkyl, Alkenyl oder Alkinyl, für Alkylcarbonyl, welches gegebenenfalls durch Halogen oder Alkoxy substituiert ist, für Benzoyl, welches gegebenenfalls durch Halogen, Nitro, Alkyl, Halogenalkyl und/oder Alkoxy substituiert ist, für Alkoxycarbonyl, für Alkylaminocarbonyl, welches gegebenenfalls durch Halogen oder Alkoxy substituiert ist, für Dialkylaminocarbonyl, für Benzyloxycarbonyl, für Benzylaminocarbonyl, welches gegebenenfalls durch Halogen, Alkyl, Halogenalkyl und/oder Alkoxy substituiert ist, oder für Phenethylaminocarbonyl, welches gegebenenfalls durch Halogen, Alkyl, Halogenalkyl und/oder Alkoxy substituiert ist, steht und

$A^2$ für Alkyl steht.

Man erhält die neuen Verbindungen der Formel (Ia), wenn man

(a) nitrosubstituierte Benzthiazolone der allgemeinen Formel (II)

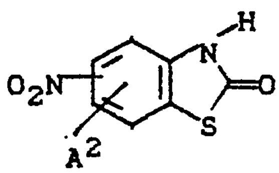

(II)

in welcher

$A^2$ die oben angegebene Bedeutung hat,

- oder Alkalimetallsalze von Verbindungen der allgemeinen Formel (II) -

mit Halogenverbindungen der allgemeinen Formel (III)

$X^1$-$A^1$ (III)

in welcher

$A^1$ die oben angegebene Bedeutung hat und

$X^1$ für Halogen steht,

gegebenenfalls in Gegenwart eines Säureakzeptors und gegebenenfalls in Gegenwart eines Verdünnungsmittels umsetzt, oder wenn man

(b) nitrosubstituierte Benzthiazol-3-oxide der allgemeinen Formel (IV)

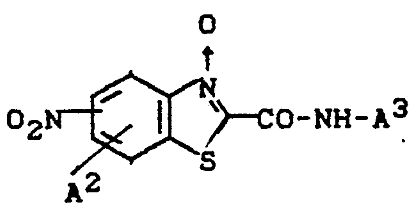

(IV)

in welcher

$A^2$ die oben angegebene Bedeutung hat und

$A^3$ für gegebenenfalls durch Halogen oder Alkoxy substituiertes Alkyl, für Benzyl, welches gegebenenfalls durch Halogen, Alkyl, Halogenalkyl und/oder Alkoxy substituiert ist, oder für Phenethyl, welches gegebenenfalls durch Halogen, Alkyl, Halogenalkyl und/oder Alkoxy substituiert ist, steht

mit Phosphorylchlorid ($POCl_3$) gegebenenfalls in Gegenwart eines Verdünnungsmittels umsetzt.

Auf prinzipiell gleiche Weise können auch die neuen und bekannten Verbindungen der allgemeinen Formel (I) hergestellt werden.

Bevorzugt werden die neuen nitrosubstituierten Benzthiazolone der Formel (Ia), in welcher

$A^1$ für $C_1$-$C_{10}$-Alkyl $C_2$-$C_{10}$-Alkenyl, $C_2$-$C_{10}$-Alkinyl, für $C_1$-$C_6$-Alkyl-carbonyl, welches gegebenenfalls ein- bis fünffach, gleich oder verschieden durch Fluor, Chlor oder $C_1$-$C_4$-Alkoxy substituiert ist, für Benzoyl, welches gegebenenfalls durch 1 bis 3, gleiche oder verschiedene Reste aus der Reihe Fluor, Chlor, Brom,

Nitro, Methyl, Ethyl, Trifluormethyl, Methoxy und Ethoxy substituiert ist, für $C_1$-$C_6$-Alkoxy-carbonyl, für $C_1$-$C_6$-Alkylaminocarbonyl, welches gegebenenfalls ein- bis dreifach, gleich oder verschieden durch Fluor, Chlor oder $C_1$-$C_4$-Alkoxy substituiert ist, für Di-($C_1$-$C_4$-alkyl)amino-carbonyl, für Benzyloxy-carbonyl, für Benzylaminocarbonyl, welches gegebenenfalls ein-bis dreifach, gleich oder verschieden durch Fluor, Chlor, Brom, Methyl, Ethyl, Trifluormethyl, Methoxy und Ethoxy substituiert ist, oder für Phenethylaminocarbonyl, welches gegebenenfalls ein- bis dreifach, gleich oder verschieden durch Fluor, Chlor, Brom, Methyl, Ethyl, Trifluormethyl, Methoxy und Ethoxy substituiert ist, steht und

$A^2$ für $C_1$-$C_4$-Alkyl steht.

Besonders bevorzugt werden die neuen Verbindungen der Formel (Ia), in welcher

$A^1$ für Methyl, Ethyl, Propyl, Isopropyl, Butyl, Isobutyl, Pentyl, Isopentyl, Hexyl oder Isohexyl, für Allyl oder Propargyl, für Acetyl, Propionyl, n-und iso-Butyroyl, n-, iso-, sec.- und tert.-Butyl-carbonyl, n-, iso-, sec.- und tert.-Pentylcarbonyl, n-, iso-, sec.- und tert.-Hexyl-carbonyl, Trifluoracetyl, Chloracetyl, Dichloracetyl, Trichloracetyl, Bromacetyl, $\alpha$- und $\beta$-Chlorpropionyl, Methoxyacetyl, Ethoxyacetyl, für Benzoyl, welches gegebenenfalls durch 1 oder 2, gleiche oder verschiedene Reste aus der Reihe Fluor, Chlor, Brom, Nitro und Methyl substituiert ist, für Methoxycarbonyl, Ethoxycarbonyl, n- oder iso-Propoxy carbonyl, n-, iso- oder sec.-Butoxycarbonyl, für Methylaminocarbonyl, Ethylaminocarbonyl, n- oder iso-Propylamino-carbonyl, n-, iso-, sec.- oder tert.-Butylamino-carbonyl, Pentylaminocarbonyl, für Dimethylaminocarbonyl oder Diethylaminocarbonyl, für Benzyloxycarbonyl, für Benzylaminocarbonyl, welches gegebenenfalls durch 1 oder 2, gleiche oder verschiedene Reste aus der Reihe Fluor, Chlor, Methyl, Trifluormethyl und Methoxy substituiert ist, oder für Phenethylaminocarbonyl, welches gegebenenfalls durch 1 oder 2, gleiche oder verschiedene Reste aus der Reihe Fluor, Chlor, Methyl, Trifluormethyl und Methoxy im Phenyl-und/oder Alkylrest substituiert ist, steht und

$A^2$ für Methyl steht.

Verwendet man als Ausgangsstoffe beim Verfahren (a) zur Herstellung der neuen und bekannten Verbindungen der Formeln (Ia) bzw. (I) beispielsweise 5-Methyl-7-nitrobenzthiazolon und 4-Fluor-benzylchlorid, so kann der Reaktionsablauf durch das folgende Formelschema wiedergegeben werden:

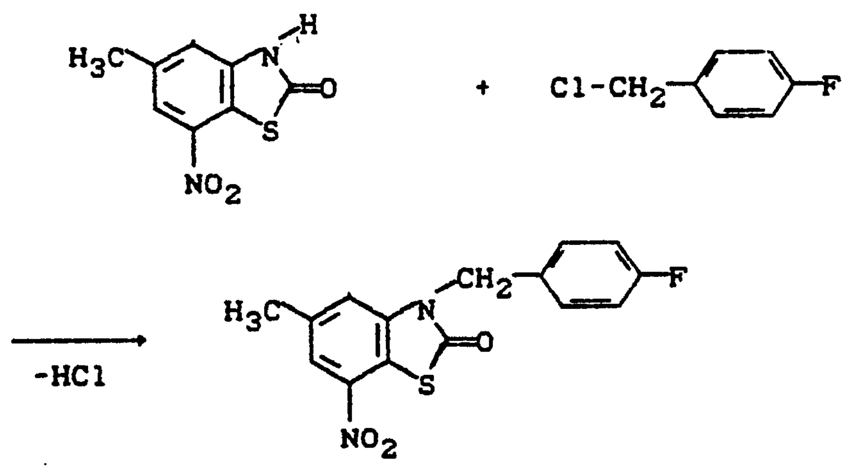


Verwendet man als Ausgangsstoffe beim Verfahren (b) zur Herstellung der neuen oder bekannten Verbindungen der Formeln (Ia) bzw. (I) beispielsweise 5-Methyl-7-nitro-2-phenethylamino-carbonylbenzthiazol-3-oxid und Phosphorylchlorid, so kann der Rekationsablauf durch das folgende Formelschema wiedergegeben werden:

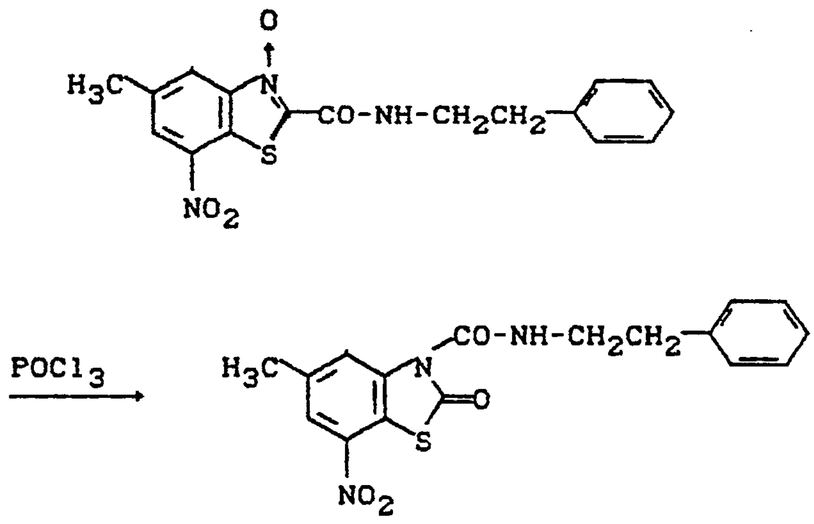


Die beim erfindungsgemäßen Verfahren (a) zur Herstellung von Verbindungen der Formel (Ia) als Ausgangsstoffe zu verwendenden nitrosubstituierten Benzthiazolone sind durch die Formel (II) allgemein definiert.

In der Formel (II) hat $A^2$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $A^2$ angegeben wurde.

Als Beispiele für die Ausgangsstoffe der Formel (II) seien genannt:
4-Methyl-5-nitro-, 4-Methyl-6-nitro-, 4-Methyl-7-nitro-, 5-Methyl-4-nitro-, 5-Methyl-6-nitro-, 5-Methyl-7-nitro-, 6-Methyl-4-nitro-, 6-Methyl-5-nitro-, 6-Methyl-7-nitro-, 7-Methyl-4-nitro-, 7-Methyl-5-nitro- und 7-Methyl-6-nitro-benzthiazolon.

Die Ausgangsstoffe der Formel (II) sind zum Teil bekannt oder können nach an sich bekannten Verfahren hergestellt werden (vgl. Egypt. J. Chem. 16 (1973), 355-359).

Neu sind die Ausgangsstoffe der Formel (IIa)

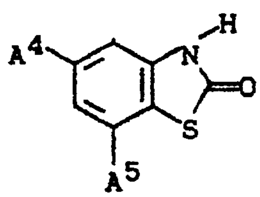
(IIa)

in welcher
jeweils der eine der beiden Substituenten $A^4$ bzw. $A^5$ für Methyl steht und der andere der beiden Substituenten $A^5$ bzw. $A^4$ für Nitro steht.

Die Ausgangsstoffe der Formeln (II) und (IIa) gehören zu den Wirkstoffen, welche durch die allgemeine Formel (I) definiert sind und deren Verwendung erfindungsgemäß ist.

Man erhält die neuen Ausgangsstoffe der Formel (IIa), wenn man entsprechende 2-Carbamoyl-benzthiazol-3-oxide der Formel (V)

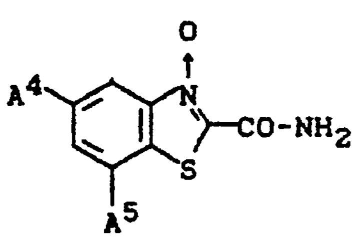
(V)

in welcher
$A^4$ und $A^5$ die oben angegebenen Bedeutungen haben,
mit Phosphorylchlorid ($POCl_3$), gegebenenfalls in Gegenwart eines Verdünnungsmittels, wie z.B. Toluol, bei Temperaturen zwischen 80 °C und 120 °C umsetzt.

Die 2-Carbamoyl-benzthiazol-3-oxide der Formel (V) sind ebenfalls noch nicht aus der Literatur bekannt. Man erhält die neuen Verbindungen der Formel (V), wenn man entsprechende 2-Alkoxycarbonyl-benzthiazol-3-oxide der Formel (VI)

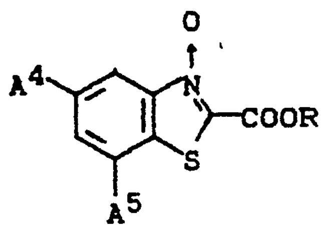

(VI)

in welcher
$A^4$ und $A^5$ die oben angegebenen Bedeutungen haben und
R für Alkyl, vorzugsweise mit 1 bis 4 Kohlenstoffatomen, steht,
mit Ammoniak in Gegenwart eines Verdünnungsmittels, wie z.B. Wasser und/oder Methanol, bei Temperaturen zwischen 0 °C und 50 °C umsetzt.

Die Zwischenprodukte der Formel (VI) können auch zur Herstellung von Ausgangsstoffen der Formel (IV) für das erfindungsgemäße Verfahren (b) verwendet werden. Die Herstellung dieser und ähnlicher Zwischenprodukte wird weiter unten im Zusammenhang mit der Beschreibung der Ausgangsstoffe für Verfahren (b) beschrieben.

Die bei Verfahren (a) weiter als Ausgangsstoffe zu verwendenden Verbindungen sind durch die Formel (III) allgemein definiert.

In Formel (III) hat $A^1$ vorzugsweise bzw. insbesondere diejenige Bedeutung, die bereits oben im Zusammenhang mit der Beschreibung der erfindungsgemäßen Verbindungen der Formel (Ia) vorzugsweise bzw. als insbesonders bevorzugt für $A^1$ angegeben wurde und $X^1$ steht vorzugsweise für Chlor, Brom oder Iod.

Als Beispiele für die Ausgangsstoffe der Formel (III) seien genannt:
Methyl-, Ethyl-, Propyl-, Butyl-, Pentyl- und Hexylchlorid, -bromid und -iodid, Isopropyl-, Isobutyl-, Isopentyl- und Isohexylchlorid und -bromid, Allyl- und Propargyl-chlorid und -bromid, Acetyl-, Propionyl-, n-und iso-Butyroylchlorid, Pivaloylchlorid, Trichloracetylchlorid, Methoxyacetylchlorid, Benzoylchlorid, 2-Fluor-, 3-Fluor-, 4-Fluor-, 2-Chlor-, 3-Chlor-, 4-Chlor-, 2-Brom-, 2-Nitro-, 4-Nitro- und 4-Methylbenzoylchlorid, Chlorameisensäure-methylester und -ethylester und Dimethylcarbamidsäurechlorid.

Die Ausgangsstoffe der Formel (III) sind bekannte organische Synthesechemikalien.

Das erfindungsgemäße Verfahren (a) zur Herstellung der neuen oder bekannten Verbindungen der Formeln (Ia) bzw. (I) wird vorzugsweise unter Verwendung von Verdünnungsmitteln durchgeführt. Als Verdünnungsmittel kommen dabei Wasser und praktisch alle inerten organischen Lösungsmittel in Frage. Hierzu gehören vorzugsweise aliphatische und aromatische, gegebenenfalls halogenierte Kohlenwasserstoffe die Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester oder Nitrile wie z.B. Acetonitril und Propionitril.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kaliummethylat, -ethylat und -tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen -20 °C und +80 °C, vorzugsweise bei Temperaturen zwischen 0 °C und 40 °C.

Das erfindungsgemäße Verfahren (a) wird im allgemeinen unter Normaldruck durchgeführt.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Die beim erfindungsgemäßen Verfahren (b) zur Herstellung von Verbindungen der Formeln (Ia) bzw. (I) als Ausgangsstoffe zu verwendenden nitrosubstituierten Benzthiazol-3-oxide sind durch die Formel (IV) allgemein definiert.

In der Formel (IV) hat $A^2$ vorzugsweise bzw. insbesondere diejenige Bedeutung, die bereits oben im Zusammenhang mit der Beschreibung der erfindungsgemäßen Verbindungen der Formel (Ia) vorzugsweise bzw. als insbesondere bevorzugt für $A^2$ angegeben wurde und

$A^3$ steht vorzugsweise für geradkettiges oder verzweigtes Alkyl mit 1 bis 6 Kohlenstoffatomen, welches gegebenenfalls ein- bis dreifach, gleich oder verschieden durch Fluor, Chlor oder $C_1$-$C_4$-Alkoxy substituiert ist, für Benzyl, welches gegebenenfalls ein- bis dreifach, gleich oder verschieden durch Fluor, Chlor, Brom, Methyl, Ethyl, Trifluormethyl, Methoxy und Ethoxy substituiert ist, oder für Phenethyl, welches gegebenenfalls ein- bis dreifach, gleich oder verschieden durch Fluor, Chlor, Brom, Methyl, Ethyl, Trifluormethyl, Methoxy und Ethoxy substituiert ist; insbesondere für Methyl, Ethyl, Propyl, Isopropyl, Butyl, Isobutyl, sec-Butyl, tert.-Butyl, Benzyl oder Phenethyl, welche gegebenenfalls ein- oder zweifach, gleich oder verschieden durch Fluor, Chlor, Methyl, Trifluormethyl und Methoxy substituiert sind.

Beispiele für die Ausgangsstoffe der Formel (IV) sind in der nachstehenden Tabelle 2 aufgeführt.

Tabelle 2

Beispiele für die Ausgangsstoffe der Formel (IV)

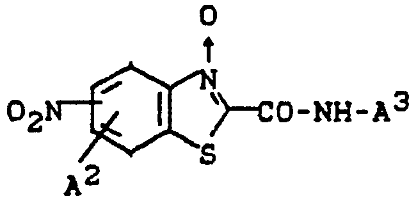

(IV)

| Position von $NO_2$ | (Position-) $A^2$ | $A^3$ |
|---|---|---|
| 5 | (7-)$CH_3$ | $CH_3$ |
| 7 | (5-)$CH_3$ | $CH_3$ |
| 5 | (7-)$CH_3$ | $C_2H_5$ |
| 7 | (5-)$CH_3$ | $C_2H_5$ |
| 5 | (7-)$CH_3$ | $C_3H_7$ |
| 7 | (5-)$CH_3$ | $C_3H_7$ |
| 5 | (7-)$CH_3$ | $C_4H_9$ |
| 7 | (5-)$CH_3$ | $C_4H_9$ |
| 5 | (7-)$CH_3$ | $C_5H_{11}$ |
| 7 | (5-)$CH_3$ | $C_5H_{11}$ |
| 5 | (7-)$CH_3$ | $C_6H_{13}$ |
| 7 | (5-)$CH_3$ | $C_6H_{13}$ |
| 5 | (7-)$CH_3$ | $CH_2-CH(CH_3)_2$ |
| 7 | (5-)$CH_3$ | $CH_2-CH(CH_3)_2$ |
| 5 | (7-)$CH_3$ | $C(CH_3)_3$ |
| 7 | (5-)$CH_3$ | $CH(CH_3)-CH_2-CH_3$ |

Tabelle 2 (Fortsetzung)

| Position von $NO_2$ | (Position-) $A^2$ | $A^3$ |
|---|---|---|
| 5 | (7-)$CH_3$ | 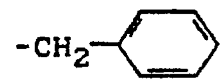 |
| 7 | (5-)$CH_3$ | 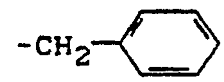 |
| 5 | (7-)$CH_3$ | 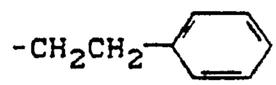 |
| 7 | (5-)$CH_3$ | 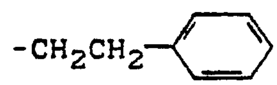 |
| 5 | (7-)$CH_3$ | 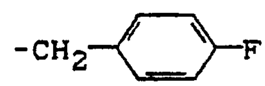 |
| 7 | (5-)$CH_3$ | 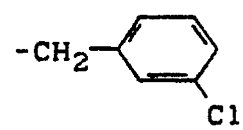 |
| 5 | (7-)$CH_3$ | 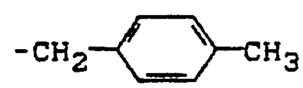 |
| 7 | (5-)$CH_3$ | 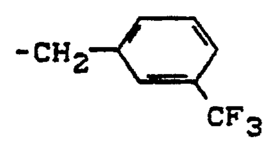 |
| 5 | (7-)$CH_3$ | 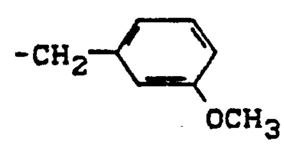 |

Tabelle 2 (Fortsetzung)

| Position von $NO_2$ | (Position-) $A^2$ | $A^3$ |
|---|---|---|
| 7 | (5-)$CH_3$ | 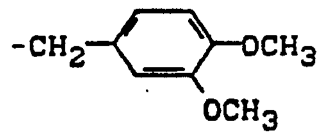 |
| 5 | (7-)$CH_3$ | 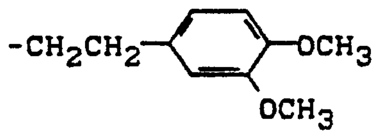 |
| 7 | (5-)$CH_3$ | 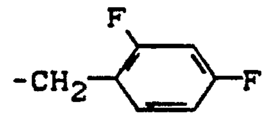 |
| 5 | (7-)$CH_3$ | 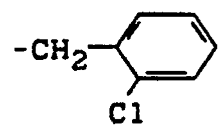 |

Die Ausgangsstoffe der Formel (IV) sind noch nicht aus der Literatur bekannt.

Man erhält die neuen nitrosubstituierten Benzthiazol-3-oxide der allgemeinen Formel (IV), wenn man entsprechende 2-Alkoxycarbonyl-benzthiazol-3-oxide der allgemeinen Formel (VIa)

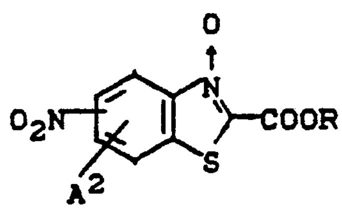

(VIa)

in welcher
$A^2$ die oben angegebene Bedeutung hat und
R für Alkyl, vorzugsweise mit 1 bis 4 Kohlenstoffatomen, steht,
mit Aminen der allgemeinen Formel (VII)

$H_2N - A^3$ (VII)

in welcher
$A^3$ die oben angegebene Bedeutung hat,
gegebenenfalls in Gegenwart von Verdünnungsmitteln, wie z.B. Methanol oder Ethanol, bei Temperaturen zwischen 0 °C und 100 °C umsetzt.

Die als Zwischenprodukte benötigten 2-Alkoxycarbonylbenzthiazol-3-oxide der allgemeinen Formel (VIa) sind ebenfalls noch nicht aus der Literatur bekannt.

Man erhält die neuen 2-Alkoxycarbonyl-benzthiazol-3-oxide der allgemeinen Formel (VIa), wenn man entsprechende 2-Chlor-nitrobenzol-Derivate der allgemeinen Formel (VIII)

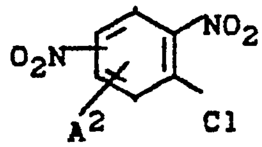

(VIII)

in welcher
$A^2$ die oben angegebene Bedeutung hat,
mit Mercaptoessigsäureestern der allgemeinen Formel (IX)
$HS - CH_2 - COOR$ (IX)
in welcher
R die oben angegebene Bedeutung hat,
in Gegenwart einer Base, wie z.B. Triethylamin, und in Gegenwart eines Verdünnungsmittels, wie z.B. Dimethylsulfoxid, Benzol, Toluol, Tetrahydrofuran, Dioxan, Methanol, Ethanol, Propanol, Isopropanol und/oder Wasser, bei Temperaturen zwischen 0° C und 100° C, vorzugsweise zwischen 20° C und 80° C, umsetzt.

Die Ausgangsstoffe der Formeln (VII), (VIII) und (IX) sind bekannte organische Synthesechemikalien.

Das erfindungsgemäße Verfahren (b) zur Herstellung der neuen Verbindungen der Formel (Ia) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methylisobutylketon, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Die Reaktionstemperaturen können bei dem erfindungsgemäßen Verfahren (b) in einem größeren Bereich variiert werden. Im allgemeinen arbeitet man bei Temperaturen zwischen 0° C und 150° C, vorzugsweise bei Temperaturen zwischen 20° C und 100° C.

Das erfindungsgemäße Verfahren (b) wird im allgemeinen bei Normaldruck durchgeführt.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren b) jeweils nach üblichen Methoden.

Die erfindungsgemäßen Wirkstoffe weisen eine starke biologische Wirkung auf und können zur Bekämpfung von unerwünschten Schädlingen praktisch eingesetzt werden. Die Wirkstoffe sind z.B. für den Gebrauch als Pflanzenschutzmittel geeignet, vor allem zur Bekämpfung von Pilzen.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus

(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Verbindungen der Formel (I) zeigen insbesondere starke protektive Wirkung gegen Pyricularia-Arten, wie z.B. Pyricularia oryzae, welche im Reisanbau Schäden verursachen, sowie gegen Venturia-Arten, wie z.B. Venturia inaequalis, welche im Obstbau Schäden hervorrufen.

Auch gegen Plasmopara- und Fusarium-Arten wird eine gute Wirkung beobachtet.

Zum Teil zeigen die Verbindungen der Formel (I) auch insektizide und akarizide Wirkung.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten

Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Anwendungsbeispiele

In den folgenden Anwendungsbeispielen wurden die nachstehend aufgeführten Verbindungen als Vergleichssubstanzen eingesetzt:

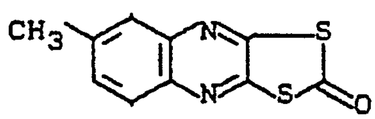

6-Methyl-2,3-chinoxalindithiol-cyclocarbonat (Chinomethionat) (vgl. DE-AS 1 100 372),

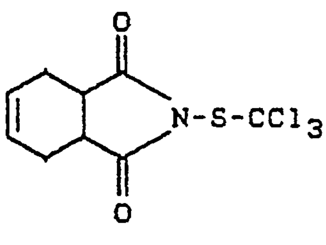

N-Trichlormethylthio-tetrahydrophthalimid (Captan) (vgl. US-P 2 553 770).

Beispiel A

Pyricularia-Test (Reis) /protektiv

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die in Tabelle 1 als Beispiele Nr. 1, 2, 4, 5, 11, 13, 17, 23, 26, 27, 31 und 32 aufgeführten Verbindungen.

## Tabelle A

Pyricularia-Test (Reis) / protektiv

| Wirkstoffe | Wirkstoffkonzentration in % | Wirkungsgrad in % der unbehandelten Kontrolle |
|---|---|---|
| 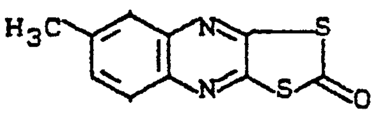 (bekannt)<br>(Chinomethionat) | 0,025 | 0 |
| 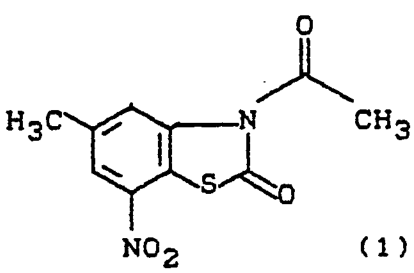 (1) | 0,025 | 89 |
| 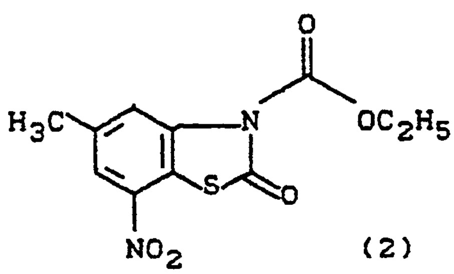 (2) | 0,025 | 89 |
| 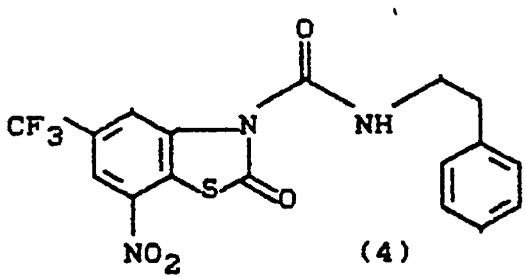 (4) | 0,025 | 100 |

Tabelle A (Fortsetzung)

Pyricularia-Test (Reis) / protektiv

| Wirkstoffe | Wirkstoffkonzentration in % | Wirkungsgrad in % der unbehandelten Kontrolle |
|---|---|---|
| 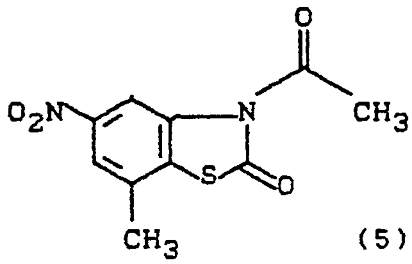 (5) | 0,025 | 90 |
| 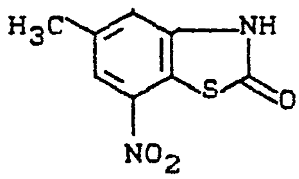 (11) | 0,025 | 100 |
| 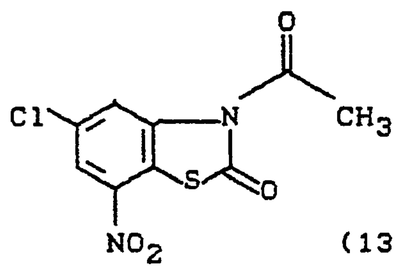 (13) | 0,025 | 89 |
| 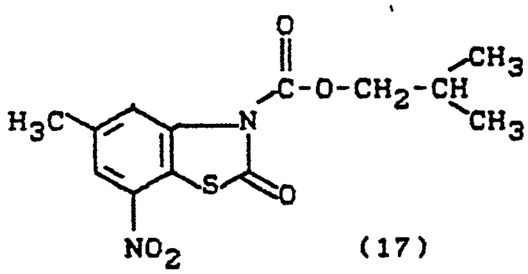 (17) | 0,025 | 89 |

Tabelle A (Fortsetzung)

Pyricularia-Test (Reis) / protektiv

| Wirkstoffe | Wirkstoffkonzentration in % | Wirkungsgrad in % der unbehandelten Kontrolle |
|---|---|---|
| 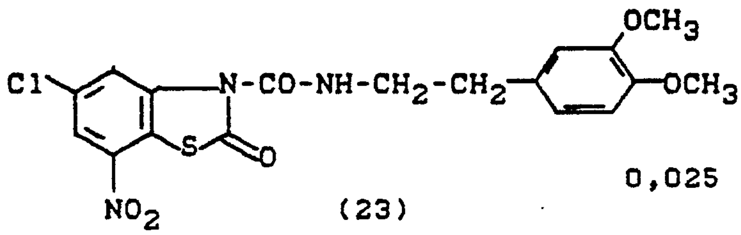 (23) | 0,025 | 80 |
| 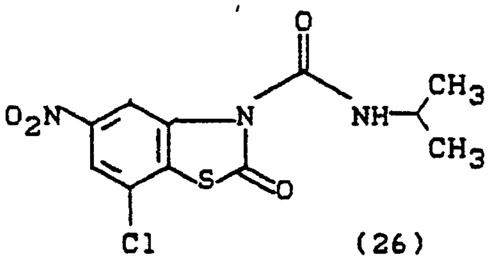 (26) | 0,025 | 90 |
| 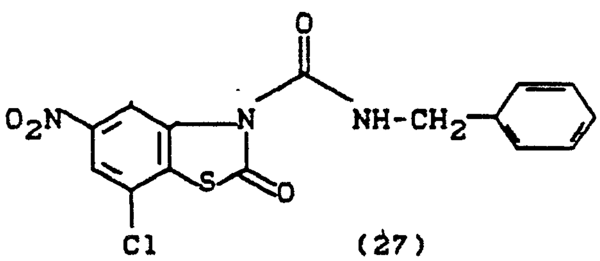 (27) | 0,025 | 90 |
| 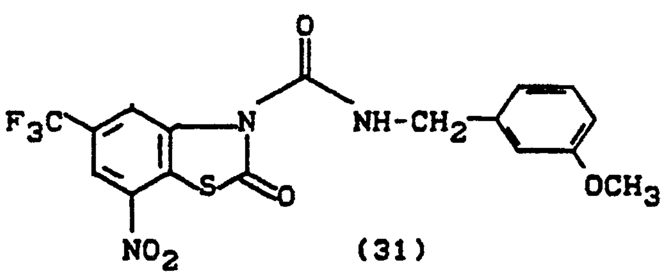 (31) | 0,025 | 90 |

Tabelle A (Fortsetzung)

Pyricularia-Test (Reis) / protektiv

| Wirkstoffe | Wirkstoffkonzentration in % | Wirkungsgrad in % der unbehandelten Kontrolle |
|---|---|---|
| 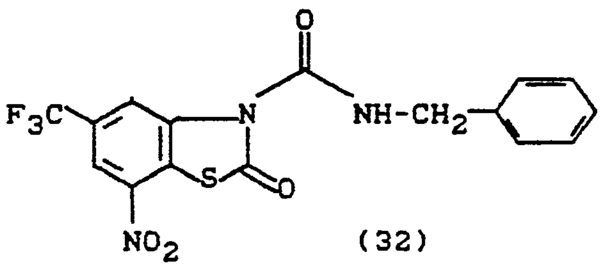 (32) | 0,025 | 90 |

Beispiel B

Venturia-Test (Apfel) / protektiv

Lösungmittel: 4,7 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20° C und 100 % relativer Luftfeuchtigkeit in einer Inkubationskabine.

Die Pflanzen werden dann im Gewächshaus bei 20° C und einer relativen Luftfeuchtigkeit von ca. 70 % aufgestellt.

12 Tage nach der Inokulation erfolgt die Auswertung.

Eine deutliche Überlegenheit in der Wirkamkeit gegenüber dem Stand der Technik zeigen in diesem Test z.B. die in Tabelle 1 als Beispiele Nr. 1, 6, 13 und 14 aufgeführten Verbindungen.

## T a b e l l e  B

Venturia -Test (Apfel) / protektiv

| Wirkstoffe | Wirkungsgrad in % der unbehandelten Kontrolle bei einer Wirkstoff-Konzentration von 5 ppm |
|---|---|
| bekannt | |
| 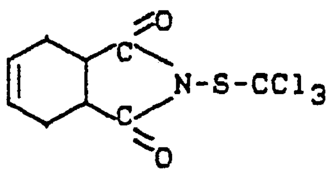 | 51 |
| erfindungsgemäß | |
| 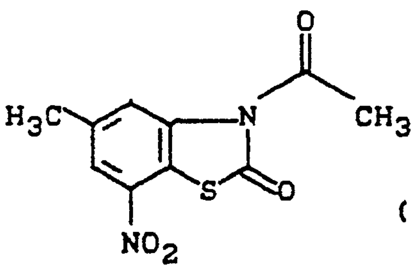 (1) | 85 |
| 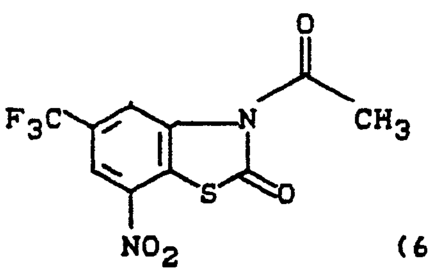 (6) | 100 |

Tabelle B (Fortsetzung)

Venturia -Test (Apfel) / protektiv

| Wirkstoffe | Wirkungsgrad in % der unbehandelten Kontrolle bei einer Wirkstoff-Konzentration von 5 ppm |
|---|---|
| erfindungsgemäß | |
| 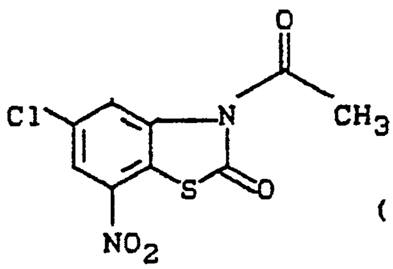 (13) | 69 |
| 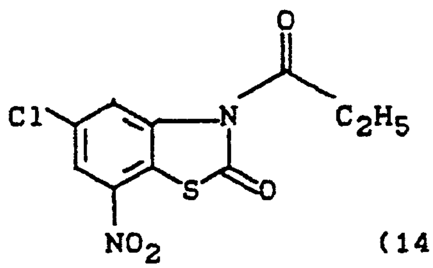 (14) | 82 |

Herstellungsbeispiele

Beispiel 1:

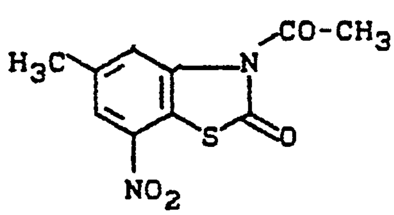

(Verfahren (a))

6,3 g (0,03 Mol) 5-Methyl-7-nitro-benzthiazolon und 3,3 g (0,03 Mol) Triethylamin werden in 150 ml Aceton gelöst. Bei 5-10 °C Innentemperatur läßt man langsam unter Rühren 2,35 g (0,03 Mol) Acetylchlorid

zutropfen. Der Reaktionsansatz wird 3 Stunden bei Raumtemperatur nachgerührt und anschließend auf Eis gegossen. Man gewinnt 6,3 g (83 % der Theorie) 3-Acetyl-5-methyl-7-nitro-benzthiazolon in Form cremefarbener Blättchen vom Schmelzpunkt 141 °C.

Analog Beispiel 1 sowie entsprechend der allgemeinen Beschreibung der erfindungsgemäßen Herstellungsverfahren (a) und (b) können auch die weiteren in Tabelle 1 aufgeführten Verbindungen der Formel (I) bzw. der Formel (Ia) hergestellt werden.

Ausgangsstoffe der Formel (IIa)

Beispiel (II a-1)

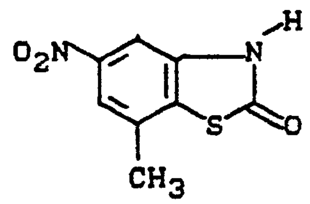

25,3 g (0,1 Mol) 2-Carbamyl-5-nitro-7-methyl-benzthiazolon-N-oxid werden in 150 ml wasserfreiem Toluol suspendiert. Zu dieser Suspension tropft man bei 100 °C Innentemperatur unter Rühren 16,8 g (0,11 Mol) Phosphoroxychlorid, kocht das Reaktionsgemisch noch 4 Stunden unter Rückfluß und läßt erkalten. Man gewinnt 15,8 g (75 % der Theorie) 5-Nitro-7-methyl-benzthiazolon vom Schmelzpunkt > 250 °C.

Analog erhält man:

Beispiel (II a-2)

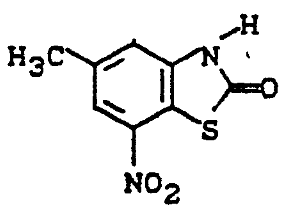

**5-Methyl-7-nitro-benzthiazolon.**

Ausgangsstoffe der Formel (V)

Beispiel (V-1)

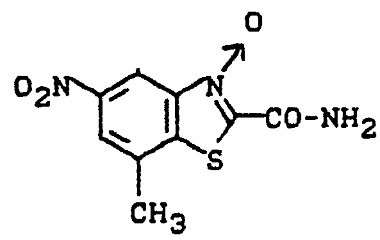

160 g (0,57 Mol) 2-Ethoxycarbonyl-5-nitro-7-methylbenzthiazol-N-oxid werden in 1,0 l Methanol vorgelegt mit 42 g (0,62 Mol $NH_3$) einer wäßrigen Ammoniaklösung versetzt und 5 Stunden bei 20° C bis 25° C gerührt. Das kristalline Produkt wird durch Absaugen isoliert. Man erhält 140 g (96 % der Theorie) 2-Carbamyl-5-nitro-7-methyl-benzthiazol-N-oxid vom Schmelzpunkt > 250° C.

## Ansprüche

1. Verwendung von nitrosubstituierten Benzthiazolonen der allgemeinen Formel (I)

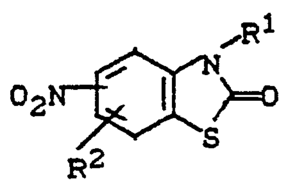

(I)

in welcher
$R^1$ für Wasserstoff, für Alkyl, Alkenyl oder Alkinyl, für Cycloalkyl-alkyl, für Furylalkyl, für Alkylcarbonyl, welches gegebenenfalls durch Halogen oder Alkoxy substituiert ist, für Benzoyl, welches gegebenenfalls durch Halogen, Nitro, Alkyl, Halogenalkyl und/oder Alkoxy substituiert ist, für Alkoxycarbonyl, für Phenoxycarbonyl, für Alkylaminocarbonyl, welches gegebenenfalls durch Halogen oder Alkoxy substituiert ist, für Dialkylaminocarbonyl, für Benzyloxycarbonyl, für Benzylaminocarbonyl, welches gegebenenfalls durch Halogen, Alkyl, Halogenalkyl und/oder Alkoxy substituiert ist, oder für Phenethylaminocarbonyl, welches gegebenenfalls durch Halogen, Alkyl, Halogenalkyl und/oder Alkoxy substituiert ist, steht und
$R^2$ für Wasserstoff, Halogen, Alkyl oder Halogenalkyl steht,
zur Bekämpfung von Pilzen.

2. Verwendung von nitrosubstituierten Benzthiazolonen der Formel (I) gemäß Anspruch 1,
in welcher
$R^1$ für Wasserstoff, für $C_1$-$C_{10}$-Alkyl, $C_2$-$C_{10}$-Alkenyl, $C_2$-$C_{10}$-Alkinyl, für Cyclohexylmethyl, für Furylmethyl, für $C_1$-$C_6$-Alkyl-carbonyl, welches gegebenenfalls ein- bis fünffach, gleich oder verschieden durch Fluor, Chlor oder $C_1$-$C_4$-Alkoxy substituiert ist, für Benzoyl, welches gegebenenfalls durch 1 bis 3, gleiche oder verschiedene Reste aus der Reihe Fluor, Chlor, Brom, Nitro, Methyl, Ethyl, Trifluormethyl, Methoxy und Ethoxy substituiert ist, für $C_1$-$C_6$-Alkoxy-carbonyl, für Phenoxycarbonyl, für $C_1$-$C_6$-Alkylaminocarbonyl, welches gegebenenfalls ein- bis dreifach, gleich oder verschieden durch Fluor, Chlor oder $C_1$-$C_4$-Alkoxy substituiert ist, für Di-($C_1$-$C_4$-alkyl)-amino-carbonyl, für Benzyloxy-carbonyl, für Benzylaminocarbonyl, welches gegebenenfalls ein- bis dreifach, gleich oder verschieden durch Fluor, Chlor, Brom, Methyl, Ethyl, Trifluormethyl, Methoxy und Ethoxy substituiert ist, oder für Phenethylaminocarbonyl, welches gegebenenfalls ein- bis dreifach, gleich oder verschieden durch Fluor, Chlor, Brom, Methyl, Ethyl, Trifluormethyl, Methoxy und Ethoxy substituiert ist, steht und
$R^2$ für Wasserstoff, Halogen, $C_1$-$C_4$-Alkyl oder $C_1$-$C_4$-Halogenalkyl mit 1 bis 9 gleichen oder verschiedenen Halogenatomen steht, zur Bekämpfung von Pilzen.

3. Verwendung von nitrosubstituierten Benzthiazolonen der Formel (I) gemäß Anspruch 1, in welcher
$R^1$ für Wasserstoff, für Methyl, Ethyl, Propyl, Isopropyl, Butyl, Isobutyl, Pentyl, Isopentyl, Hexyl oder Isohexyl, für Allyl oder Propargyl, für Acetyl, Propionyl, n- und iso-Butyroyl, n-, iso-, sec.- und tert.-Butylcarbonyl, n-, iso-, sec.- und tert.-Pentyl-carbonyl, n-, iso-, sec.- und tert.-Hexyl-carbonyl, Trifluoracetyl, Chloracetyl, Dichloracetyl, Trichloracetyl, Bromacetyl, α- und β-Chlorpropionyl, Methoxyacetyl, Ethoxyacetyl, für Benzoyl, welches gegebenenfalls durch 1 oder 2, gleiche oder verschiedene Reste aus der Reihe

Fluor, Chlor, Brom, Nitro und Methyl substituiert ist, für Methoxycarbonyl, Ethoxycarbonyl, n- oder iso-Propoxycarbonyl, n-, iso- oder sec.- Butoxycarbonyl, für Methylaminocarbonyl, Ethylaminocarbonyl, n-oder iso-Propylamino-carbonyl, n-, iso-, sec.-oder tert.-Butylamino-carbonyl, Pentylaminocarbonyl, für Dimethylaminocarbonyl oder Diethylaminocarbonyl, für Benzyloxycarbonyl, für Benzylaminocarbonyl, welches gegebenenfalls durch 1 oder 2, gleiche oder verschiedene Reste aus der Reihe Fluor, Chlor, Methyl, Trifluormethyl und Methoxy substituiert ist, oder für Phenethylaminocarbonyl, welches gegebenenfalls durch 1 oder 2, gleiche oder verschiedene Reste aus der Reihe Fluor, Chlor, Methyl, Trifluormethyl und Methoxy substituiert ist, steht und

$R^2$ für Chlor, Methyl oder Trifluormethyl steht,

zur Bekämpfung von Pilzen.

4. Verfahren zur Bekämpfung von Pilzen, dadurch gekennzeichnet, daß man nitrosubstituierte Benzthiazolone der Formel (I) gemäß den Ansprüchen 1 bis 3 auf die Pilze und/oder ihren Lebensraum einwirken läßt.

5. Verfahren zur Herstellung von fungiziden Mitteln, dadurch gekennzeichnet, daß man nitrosubstituierte Benzthiazolone der Formel (I) gemäß den Ansprüchen 1 bis 3 mit Streckmitteln und/oder oberflächenaktiven Substanzen vermischt.

6. Nitrosubstituierte Benzthiazolone der allgemeinen Formel (Ia)

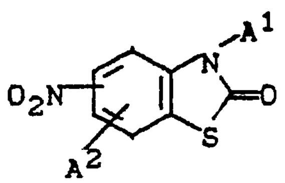

(Ia)

in welcher

$A^1$ für Alkyl, Alkenyl oder Alkinyl, für Alkylcarbonyl, welches gegebenenfalls durch Halogen oder Alkoxy substituiert ist, für Benzoyl, welches gegebenenfalls durch Halogen, Nitro, Alkyl, Halogenalkyl und/oder Alkoxy substituiert ist, für Alkoxycarbonyl, für Alkylaminocarbonyl, welches gegebenenfalls durch Halogen oder Alkoxy substituiert ist, für Dialkylaminocarbonyl, für Benzyloxycarbonyl, für Benzylaminocarbonyl, welches gegebenenfalls durch Halogen, Alkyl, Halogenalkyl und/oder Alkoxy substituiert ist, oder für Phenethylaminocarbonyl, welches gegebenenfalls durch Halogen, Alkyl, Halogenalkyl und/oder Alkoxy substituiert ist, steht und

$A^2$ für Alkyl steht.

7. Verfahren zur Herstellung von nitrosubstituierten Benzthiazolonen der Formel (Ia) gemäß Anspruch 6, dadurch geknnzeichnet, daß man

(a) nitrosubstituierte Benzthiazolone der allgemeinen Formel (II)

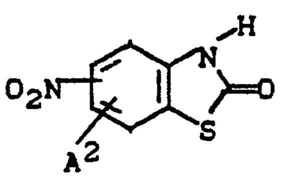

(II)

in welcher

$A^2$ die in Anspruch 6 angegebene Bedeutung hat,

- oder Alkalimetallsalze von Verbindungen der allgemeinen Formel (II) -

mit Halogenverbindungen der allgemeinen Formel (III)

$X^1$-$A^1$ (III)

in welcher

$A^1$ die in Anspruch 6 angegebene Bedeutung hat und

$X^1$ für Halogen steht,

gegebenenfalls in Gegenwart eines Säureakzeptors und gegebenenfalls in Gegenwart eines Verdünnungsmittels umsetzt, oder daß man

(b) nitrosubstituierte Benzthiazol-3-oxide der allgemeinen Formel (IV)

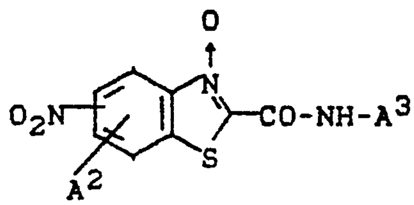

(IV)

in welcher
$A^2$ die in Anspruch 6 angegebene Bedeutung hat und
$A^3$ für gegebenenfalls durch Halogen oder Alkoxy substituiertes Alkyl, für Benzyl, welches gegebenenfalls durch Halogen, Alkyl, Halogenalkyl und/oder Alkoxy substituiert ist, oder für Phenethyl, welches gegebenenfalls durch Halogen, Alkyl, Halogenalkyl und/oder Alkoxy substituiert ist, steht
mit Phosphorylchlorid ($POCl_3$) gegebenenfalls in Gegenwart eines Verdünnungsmittels umsetzt.

8. Monomethyl-mononitrosubstituiertes Benzthiazolon der Formel (IIa)

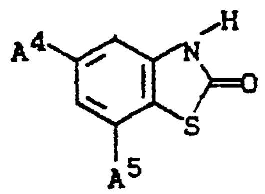

(IIa)

in welcher
jeweils der eine der beiden Substituenten $A^4$ bzw. $A^5$ für Methyl steht und der andere der beiden Substituenten $A^5$ bzw. $A^4$ für Nitro steht.

9. Verfahren zur Herstellung von monomethyl-mononitrosubstituierten Benzthiazolonen der Formel (IIa), dadurch gekennzeichnet, daß man 2-Carbamoyl-benzthiazol-3-oxide der Formel (V)

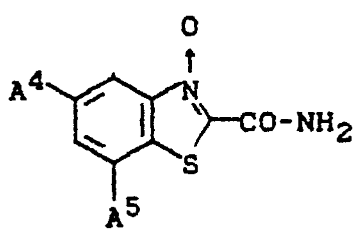

(V)

in welcher
$A^4$ und $A^5$ die in Anspruch 8 angegebenen Bedeutungen haben,
mit Phosphorylchlorid ($POCl_3$), gegebenenfalls in Gegenwart eines Verdünnungsmittels bei Temperaturen zwischen 80 °C und 120 °C umsetzt.

10. 2-Carbamoyl-benzthiazol-3-oxide der Formel (V)

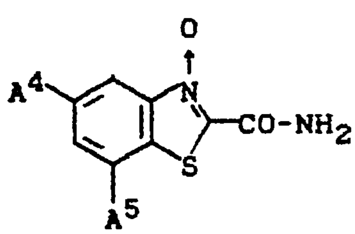

(V)

in welcher
jeweils einer der Reste $A^4$ bzw. $A^5$ für Methyl steht und der andere Rest $A^4$ bzw. $A^5$ für Nitro steht.

11. Verfahren zur Herstellung von 2-Carbamoyl-benzthiazol-3-oxiden der Formel (V) gemäß Anspruch

10, dadurch gekennzeichnet, daß man 2-Alkoxycarbonylbenzthiazol-3-oxide der Formel (VI)

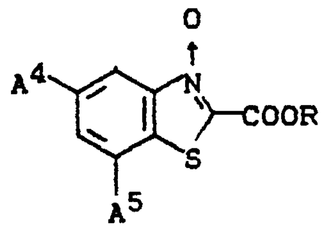

(VI)

in welcher
$A^4$ und $A^5$ die in Anspruch 10 angegebenen Bedeutungen haben und
R für Alkyl steht, mit Ammoniak in Gegenwart eines Verdünnungsmittels umsetzt.

12. Verwendung der Verbindungen der Formel (IIa), (IV) und (V) gemäß den Ansprüchen 8 und 10 als Zwischenprodukte.

13. Nitrosubstituierte Benzthiazol-3-oxide der allgemeinen Formel (IV)

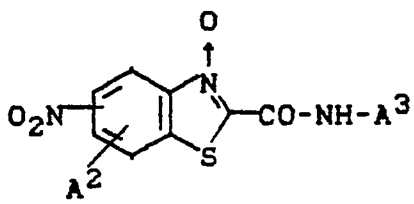

(IV)

in welcher
$A^2$ für Alkyl und
$A^3$ für gegebenenfalls durch Halogen oder Alkoxy substituiertes Alkyl, für Benzyl, welches gegebenenfalls durch Halogen, Alkyl, Halogenalkyl und/oder Alkoxy substituiert ist, oder für Phenethyl, welches gegebenenfalls durch Halogen, Alkyl, Halogenalkyl und/oder Alkoxy substituiert ist.

14. Verfahren zur Herstellung von Verbindungen der Formel (IV) gemäß Anspruch 13, dadurch gekennzeichnet, daß man 2-Alkoxycarbonyl-benzthiazol-3-oxide der allgemeinen Formel (VIa)

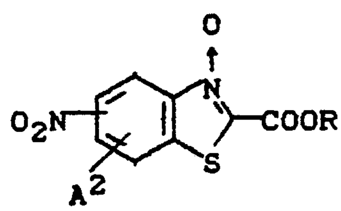

(VIa)

in welcher
$A^2$ für Alkyl und
R für Alkyl steht, mit Aminen der Formel (VII)
$H_2N\text{-}A^3$ (VII)
in welcher
$A^3$ die in Anspruch 13 angegebene Bedeutung hat,
gegebenenfalls in Gegenwart von Verdünnungsmitteln umsetzt.

Europäisches Patentamt

# EUROPÄISCHER RECHERCHENBERICHT

Nummer der Anmeldung

EP 90105656.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| A | EP - A1 - 0 218 972 (SUMITOMO CHEMICAL COMPANY, LIMITED) * Ansprüche 1,8-13; Seiten 28-31 (Tabelle 4) * -- | 1,4-14 | C 07 D 277/68<br>A 01 N 43/78 |
| A | EP - A1 - 0 296 416 (NIHON TOKUSHU NOYAKU SEIZO K.K.) * Ansprüche 1,9,10; Seite 14, Verb.(XII); Seite 15, Verb.(XIV) * -- | 1,4-14 | |
| A | CHEMICAL ABSTRACTS, Band 104, Nr. 5, 3.Februar 1986 Columbus, Ohio, USA IDEMITSU KOSAN CO.LTD. "Benzothiazolones as fungicides" Seite 216, Spalte 2, Zusammenfassung-Nr. 30 422x & Jpn. Kokai Tokkyo Koho JP 60 105 604 (85 105 604) -- | 1,4-14 | RECHERCHIERTE SACHGEBIETE (Int Cl⁵)<br>C 07 D 277/00 |
| A | CHEMICAL ABSTRACTS, Band 103, Nr. 19, 11.November 1985 Columbus, Ohio, USA IDEMITSU KOSAN CO.LTD. "Benzothiazolinones" Seite 712, Spalte 2, Zusammenfassung-Nr. 160 499t & Jpn. Kokai Tokkyo Koho JP 60 105 671 (85 105 671) ---- | 1,4-14 | |

Der vorliegende Recherchenbericht wurde für alle Patentansprüche erstellt.

| Recherchenort | Abschlußdatum der Recherche | Prüfer |
|---|---|---|
| WIEN | 14-05-1990 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument